# EUROPEAN PATENT SPECIFICATION

(11) **EP 2 855 677 B1**
(45) Date of publication and mention of the grant of the patent: **14.12.2016**
(21) Application number: 13723942.2
(22) Date of filing: 20.05.2013
(51) Int. Cl.: C12N 15/10

(54) **NUCLEIC ACID EXTRACTION**
NUKLEINSÄUREEXTRAKTION
EXTRACTION D'ACIDES NUCLÉIQUES

(30) Priority: 25.05.2012 GB 201209229
(43) Date of publication of application: 08.04.2015
(73) Proprietor: Epistem Limited, Manchester M13 9XX (GB)
(72) Inventor: COBB, Ben, North Wraxall Wiltshire SN14 8RY (GB)
(74) Representative: Williams, Gareth Owen
(86) International application number: PCT/GB2013/051301
(87) International publication number: WO 2013/175188

(56) References cited:
- TIMOFEEVA L ET AL: "Antimicrobial polymers: Mechanism of action, factors of activity, and applications", APPLIED MICROBIOLOGY AND BIOTECHNOLOGY 2011 SPRINGER VERLAG DEU, vol. 89, no. 3, February 2011 (2011-02), pages 475-492, XP002715548, ISSN: 0175-7598
- KNEUER CARSTEN ET AL: "Silica nanoparticles modified with aminosilanes as carriers for plasmid DNA", INTERNATIONAL JOURNAL OF PHARMACEUTICS (KIDLINGTON), vol. 196, no. 2, 10 March 2000 (2000-03-10), pages 257-261, XP002715549, ISSN: 0378-5173
- JUNGKYU KIM ET AL: "Microfluidic sample preparation: cell lysis and nucleic acid purification", INTEGRATIVE BIOLOGY, ROYAL SOCIETY OF CHEMISTRY, UK, vol. 1, no. 10, 1 October 2009 (2009-10-01), pages 574-586, XP008120807, ISSN: 1757-9694, DOI: 10.1039/B905844C [retrieved on 2009-08-25]
- GONG S -Q ET AL: "Quaternary ammonium silane-functionalized, methacrylate resin composition with antimicrobial activities and self-repair potential", ACTA BIOMATERIALIA 2012 ELSEVIER LTD GBR, vol. 8, no. 9, 29 May 2012 (2012-05-29), pages 3270-3282, XP002715550, ISSN: 1742-7061

## Description

### FIELD OF THE INVENTION

The present invention relates to methods and products for extraction of nucleic acids from biological samples, primarily biological samples comprising cellular material. Aspects of the invention further relate to methods for preparing nucleic acids from biological samples for nucleic acid amplification.

### BACKGROUND TO THE INVENTION

One of the main challenges for diagnostics using nucleic acid amplification technology is the pretreatment of cellular material in order to extract nucleic acids prior to the amplification process. Silica based and magnetic bead based technologies are inefficient (≤10%) and require optimisation for specific target types; the same process is generally not compatible with other sample types. High yield processes using phenol chloroform are not suited because of the toxicity of components. Detergent based methods offer a simple lysis process, but require elevated temperatures (95°C), which are not compatible with single step RT-PCR since the reverse transcriptase is not thermal stable.

Current methods involve the isolation of nucleic acids in a purified form, away from other cellular material. This generally requires breaking of cells using enzymes such as lysozyme or detergents. Mycobacterium requires a harsh NALC-NaOH pre-treatment to rupture cells. Proteins are removed by digestion using appropriate proteases (e.g. Proteinase K). Nucleic acids are bound to a support resin or charged matrix (e.g. magnetic particles) and washed. Nucleic acids are removed from the charged support through pH change into an appropriate buffer. This results in high purity nucleic acids.

State of the art systems from e.g. Cepheid, Enigma Diagnostics, Roche, Geneprobe, BD, etc integrate this conventional laboratory nucleic acid extraction process into their diagnostic instruments. This is however complex, expensive and directed to specific sample types.

We describe herein a process that removes the complexity of this process, delivering nucleic acids capable of being amplified by PCR in a simple, disposable, paper based system that works at room temperature, is capable of rupturing bacterial, fungal and viral cells, and releases nucleic acids into solution for direct-to-PCR analysis.

International patent applications WO00/62023 and WO02/16383 to Whatman, Inc, describe methods for lysing cells and isolating nucleic acids using coated filter media. The filter media is FTA-treated nitrocellulose, which is coated with an anionic detergent, for example SDS. The coating lyses the cells, and the FTA-treated filter adsorbs nucleic acids thereto. The coating is not covalently bound to the filter, and may be removed by washing. Nucleic acids can be eluted from the filter for subsequent processing.

International patent application WO2008/134464 to 3M Innovative Properties Company describes a substrate with functional groups attached, a lysing material, a matrix material, and a saccharide, for use in isolating nucleic acids from samples.

US patent application 2007/0185322 to Akhavan-Tafti describes methods for extraction of RNA from a sample involving the use of an acidic solution and a solid phase binding material which can liberate nucleic acids without performing preliminary lysis of cells. The use of quaternary ammonium salts to bind nucleic acids is mentioned. US application 2005/0042661 to Tarkkanen et al also describes use of quaternary ammonium compounds to selectively release nucleic acids from cells.

International patent applications WO2004/087226 and WO2006/071191 to Appeartex AB, and US patent 5,064,613 to Higgs et al describe antimicrobial compositions including quaternary ammonium salts.

Kim et al: "Microfluidic sample preparation: cell lysis and nucleic acid purification", Integrative Biology, Royal Society of Chemistry, UK, vol. 1, no. 10, 1 October 2009, pages 574-586, is a review describing a variety of cell lysis and nucleic acid purification techniques.

### SUMMARY OF THE INVENTION

According to a first aspect of the invention there is provided a method of preparing nucleic acids from a biological sample comprising a) cellular material having a cell membrane, or b) cellular material having a cell wall, or c) viral material having a viral envelope or d) viral material having a viral capsid; the method comprising contacting the sample with a substrate, the substrate being functionalised with a biocidal agent which is capable of i) weakening the cell membrane, cell wall, viral envelope, or viral capsid; or ii) lysing cellular or viral material; wherein the biocidal agent comprises multiple functional groups, the multiple functional groups comprising a silyl group, which is involved in binding the agent to the substrate; an alkyl chain; and an ammonium chloride group.

According to an aspect of the disclosure, there is provided a method of preparing nucleic acids from a biological sample comprising a) cellular material having a cell membrane, or b) cellular material having a cell wall, or c) viral material having a viral envelope or d) viral material having a viral capsid; the method comprising contacting the sample with a substrate, the substrate being functionalised with a biocidal agent which is capable of i) weakening the cell membrane, cell wall, viral envelope, or viral capsid; or ii) lysing cellular or viral material.

The method may further comprise the step of subjecting the sample to heat after the contacting step. For example, the sample may be directly added to a nucleic acid amplification reaction. The heat step will serve to lyse weakened cell wall, cell membrane, viral envelope, or viral capsid, to release nucleic acid. The heat step may not always be necessary to release nucleic acid, for example if the biocidal agent is capable of lysing cellular or viral material.

In this way, the method permits rapid and easy preparation of nucleic acid from a biological sample for further processing.

The substrate is preferably a cellulose material; for example, a cellulose filter paper or a cellulose matrix. The cellulose material may be a composite paper; for example, a composite cellulose paper may comprise a lateral flow layer, to remove liquid and low molecular weight contaminants and inhibitors from the sample which is deposited on a surface of the paper. Cellulose has the advantage that it has a number of exposed hydroxyl groups to which biocidal agents may be attached. In certain embodiments, the cellulose material may further comprise reagents for carrying out desired actions on the sample; for example, RNAses, proteases, and the like, for sample cleanup. A preferred substrate is a cotton-derived cellulose paper, and in particular FP 2992 paper from Hahnemühle FineArt GmbH (Germany).

Other substrate materials may be used, preferably those which have exposed hydroxyl groups. For example, the substrate may be glass, plastics, or the like. Although in a preferred embodiment the substrate is a filter paper, which may be directly added to a nucleic acid amplification reaction, in other embodiments the substrate may take the form of a microfluidic channel or a reaction vessel or other container, along which or within which the biological sample may be passed or contained.

The biocidal agent comprises multiple functional groups. The functional groups include a binding moiety, which is involved in binding the agent to the substrate; a hydrophobic moiety; and a charged moiety. The hydrophobic moiety is able to interact with and penetrate the cell wall or cell membrane. The hydrophobic moiety is an alkyl chain, for example C5-C30 alkyl, preferably C10-C20 alkyl. As the alkyl chain penetrates the delicate cell wall, the wall is weakened and punctured. The charged moiety is positively charged, and is able to attract a charged cell wall, and can disrupt ion flow and homeostasis on contacting a cell membrane, thereby helping to disrupt the cell and release the nucleic acids. The charged moiety is a quaternary ammonium group.
The functional groups are an alkyl chain (the hydrophobic moiety), a silyl group (the binding moiety), and an ammonium chloride group (the charged moiety).

Preferred biocidal agents include silylated quaternary ammonium compounds (SiQACs); in particular 3-(trimethoxysilyl) propyldimethyloctadecyl ammonium chloride (3-TPAC). Other biocidal agents of the disclosure include benzyl ammonium chlorides. The lethal mode of action of SiQACs is generally accepted to proceed by adsorption of the positively charged molecule onto the negatively charged cell surface, disruption of the cell membrane by a lipophilic chain on the SiQAC molecule, and diffusion through the membrane leading to cell lysis.

The skilled person will be aware of other suitable biocidal agents which may be used. The selection of a particular agent will be guided by the presence of the preferred functional groups described above, and the nature of the intended biological sample - for example, where the sample to be processed is a mammalian cellular sample, then there is no cell wall to penetrate, and other functional groups may be appropriate.

Examples of other biocidal agents which may be used in the present disclosure include:
a) telechelic poly(2-alkyl-1,3-oxazolines);
b) cellulose with an antimicrobial DDA group grafted via PEtOx, which kills approaching microbial cells on contact (Bieser et al (2011), Contact-Active Antimicrobial and Potentially Self-Polishing Coatings Based on Cellulose. Macromol. Biosci., 11, 111-121);
c) saponins are steroid or triterpenoid glycosides, common in a large number of plants, and have long been known to have a lytic action on erythrocyte membrane and many saponins are known to be antimicrobial (Francis et al, British Journal of Nutrition (2002), 88, 587-605). Extensive research has been carried out into the membrane-permeabilising properties of saponins. These structurally diverse compounds have also been observed to kill protozoans and to act as anti-fungal and antiviral agents. Isolated cell membranes from human erythrocytes when treated with saponin developed pores of 40-50 A° diameter as against the 80 A° pores produced in artificial membranes (Seeman et al. 1973 Structure of membrane holes in osmotic and saponin hemolysis. Journal of Cell Biology 56, 519-527).

For a review of other compositions which may be used, see "Antimicrobial Polymers in Solution and on Surfaces", Siedenbiedel and Tiller (2012) Polymers, 4, 46-71.

Preferably, the biological sample may comprise cellular material having a cell wall. For example, the cellular material may be prokaryotic cells, such as bacterial cells; or may be plant or fungal cells. Alternatively, the biological sample comprises cellular material having a cell membrane, and no cell wall. For example, the cellular material may be eukaryotic animal cells, including mammalian or insect cells. Alternatively, the biological sample may comprise viral material.

A further aspect of the present disclosure provides a method of amplifying nucleic acids in a biological sample, the method comprising: preparing nucleic acids according to the method of the above first aspect of the disclosure; and subjecting the prepared acids to a nucleic acid amplification step, preferably a polymerase chain reaction (PCR) amplification.

The amplification may be carried out for diagnostic purposes.

A further aspect of the invention provides a device
for preparing nucleic acids from a biological sample comprising a) cellular material having a cell membrane, or b) cellular material having a cell wall, or c) viral material having a viral envelope or d) viral material having a viral capsid; the device comprising a substrate functionalised with a biocidal agent which is capable of i) weakening the cell membrane, cell wall, viral envelope, or viral capsid; or ii) lysing cellular or viral material. wherein the biocidal agent comprises multiple functional groups, the multiple functional groups comprising a silyl group, which is involved in binding the agent to the substrate; an alkyl chain; and an ammonium chloride group.

The substrate is preferably a cellulose material.

In certain embodiments, the substrate may be integrated into a sample preparation cartridge or the like. Thus, the device may further comprise a reaction vessel for receiving the substrate, or a portion of the substrate. The device may yet further comprise means for separating a portion of the substrate from the remaining substrate; this may allow a piece of the substrate to be separated once the sample has been added, and the separated piece then allowed to enter the reaction vessel and used in a PCR.

### BRIEF DESCRIPTION OF THE DRAWINGS

Figure 1 shows the structure of a SiQAC molecule, 3-TPAC.
Figure 2 illustrates the functionalisation of cellulose by a SiQAC.
Figure 3 is a schematic of the mode of action of a SiQAC on a cell.
Figure 4 shows the detection of Plasmodium species from blood treated with a SiQAC.
Figure 5 compares different paper processing methods for extraction of Mycobacterium DNA.
Figure 6 shows accuracy data for clinical samples.
Figure 7 shows multiple repeat tests of MTB at different concentrations extracted from sputa using the process described, the y axis showing the peak melt temperature and the x axis showing the peak height.
Figure 8 shows the optimised process workflow for production of SiQAC functionalised paper and sample analysis.
Figure 9 shows the isolation of microbial DNA from whole dairy milk.
Figure 10 shows the amplification of nucleic acid obtained from HIV positive plasma samples.
Figure 11 shows the sequences obtained from the amplified HIV nucleic acid.

### DETAILED DESCRIPTION OF THE DRAWINGS

The present disclosure operates generally as follows. A substrate, such as cellulose filter paper, is functionalised with a biocidal agent, preferably a SiQAC, more preferably 3-TPAC. The functionalised substrate may be used in sample preparation to extract nucleic acids from a cellular sample for use in PCR reactions.

Cellular material is deposited onto the top layer of the paper. Liquid flows through the paper and the cellular material becomes trapped on the surface. The liquid phase and any low molecular weight inhibitory ions are dispersed in a secondary lateral flow layer located below the surface functionalised cellulose layer.

Microbial cells in the sample (or plant, animal, or fungal cells) interact with the SiQAC functionalised cellulose and are weakened and/or lysed, releasing their cellular content typically within 5 minutes of contact.

A plug of composite paper may be excised from the substrate, and added directly to a PCR reaction after a 10 minute period. The initial heat of the PCR can serve to additionally lyse any remaining cells to release further nucleic acid.

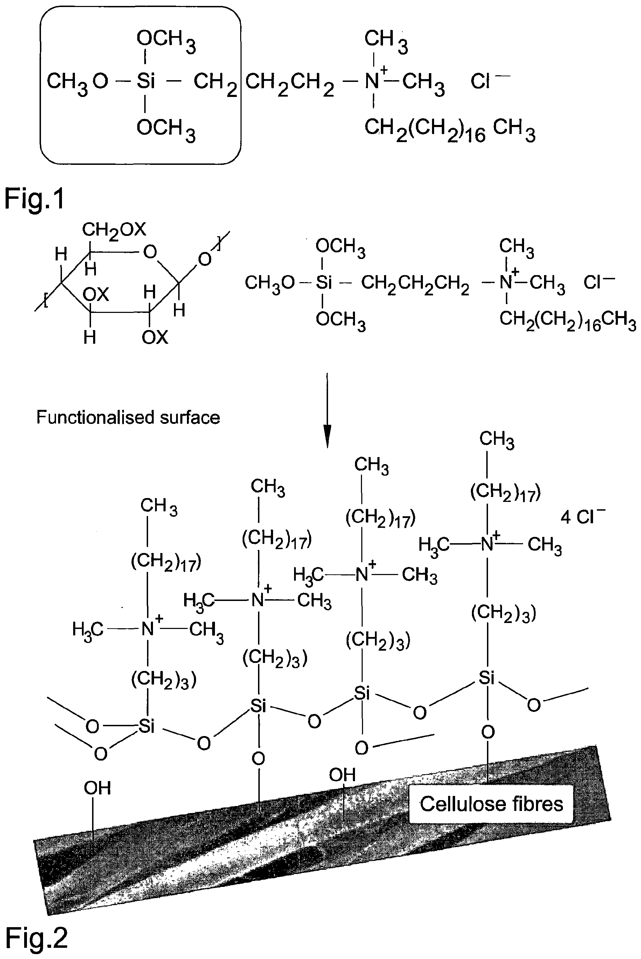

The structure of a SiQAC molecule [3-(trimethoxysilyl) propyldimethyloctadecyl ammonium chloride (3-TPAC)] is shown in Figure 1. 3-TPAC was first described in 1972 (see Isquith et al (1972), Appl. Microbiol, 24:6 859-863, http://www.ncbi.nlm.nih.gov/pmc/articles/PMC380687/pdf/applmicro00052-0033.pdf).

There are many versions of the basic chemistry (e.g. benzyl ammonium chlorides BAC), but all share similar key ingredients: ammonium chloride variant (the active antimicrobial), silicon as a binding agent (the silyl part) and an alkane chain. The ammonium chloride is a quaternary ammonium group which is attached to two methyl groups and effectively two longer chain alkyl groups. This cationic function confers antimicrobial properties which result in the breaking of bacterial, fungal and viral membranes, releasing the nucleic acid content. The hydrophobic alkane chain penetrates cell walls. The trimethoxysilyl group binds the molecule to a substrate (e.g. cellulose) via the active hydroxyl group.

Quaternary ammonium compounds are lethal to a wide variety of organisms including bacteria, fungi and coated viruses, and to a lesser extent to endospores, Mycobacterium tuberculosis and non-enveloped viruses. Many biocidal polymers are known with quaternary ammonium groups. Quaternary ammonium (QA) compounds are among the most widely used antibacterial agents for medical and public health applications, and have been shown to be effective against both gram negative and gram positive bacteria (Tashiro (2001) Macromol. Mater. Eng.; 286, 63-87).

Cationic polymers with QA groups generally exhibit higher antimicrobial activities than their corresponding low molecular weight monomers [Ikeda and Tazuke, 1983, Makromol. Chem., Rapid Commun. 4 (1983) 459-461]. The higher activity is attributed to greater electrostatic attraction between the cell and polymer due to the greater charge density of the polymer.

SiQACs work through a two-step process. The positively charged action on the SiQAC molecule attracts the negatively charged cell wall of the microorganism. Initially, the hydrophobic alkyl chain penetrates the similarly hydrophobic cell wall of an organism that it comes in contact with. As the alkyl chain penetrates the delicate cell wall, the wall is weakened and punctured. Second, as the cationic quaternary ammonium group comes in contact with the cell wall it disrupts the ion flow and causes leakage into or out of the cell wall, usually resulting in the cell losing its contents or bursting depending on the ionic environment. The charged quaternary ammonium alkyl group remains unchanged and is available to repeat the process indefinitely.

Because of this "physical" and "electrical" killing mechanism, microbes do not get an opportunity to develop resistance or immunity to the SiQAC.

Quaternary ammonium compounds are widely used as disinfectants, antiseptics, pharmaceutical products, and cosmetics and could be an alternative in fruit and vegetables disinfection. All quaternary ammonium compounds (QACs) are cationic compounds that possess a basic structure (NH4+). These compounds penetrate into the bacteria cell wall, reacting with the cytoplasmic membrane inducing wall lysis caused by autolytic enzymes (McDonnell, G. & Russell, A. D. 1999 Antiseptics and disinfectants: activity, action and resistance. Clinical Microbiology Reviews 12, 147-179).

The trimethoxysilyl groups react with hydroxyl groups on surfaces such as glass and cotton to form covalent bonds that retain the QA compound at the surface and prevent it from dissolving in water. The trimethoxysilyl groups can also react with each other to form a highly stable cross-linked silane coating bound to treated surfaces. These coatings have been shown to impart biocidal activity to surfaces in many applications without the release of chemical agents into the surrounding environment [Isquith et al, 1972; Isquith et al, 1973 U. S. Patent 3,730,701; Speier and Malek, 1982 J of Colloid and Interface Science 89, 68; Walters et al., 1973, J., Appl. Microbiol. 25, 253].

While these coatings were very effective as a fungicide and an antibacterial agent, they have been ineffective against spores.

Figure 2 illustrates the functionalisation of cellulose with a SiQAC molecule, 3-TPAC. In unaltered native cellulose, X represents hydrogen, forming a number of pendant hydroxyl (OH) groups. In the faster initial stage a bond forms between the molecule and the hydroxyl group forming a silyl ether. In the second slower step cross-links are formed between adjacent dimethoxysilyl groups to form a random silicone ether polymer aligned parallel to the substrate surface.

The cationic moiety plays no part in the surface binding but is available yet bound to the substrate surface. Its structure is analogous to the quaternary ammonium compounds recognised as topical antiseptics of which didecyldimethylammonium chloride (DDAC) is a typical example.

The mechanism whereby the SiQAC molecule becomes bound to the substrate surface is similar chemically to that in the cross-linking of polyethene to form PEX.

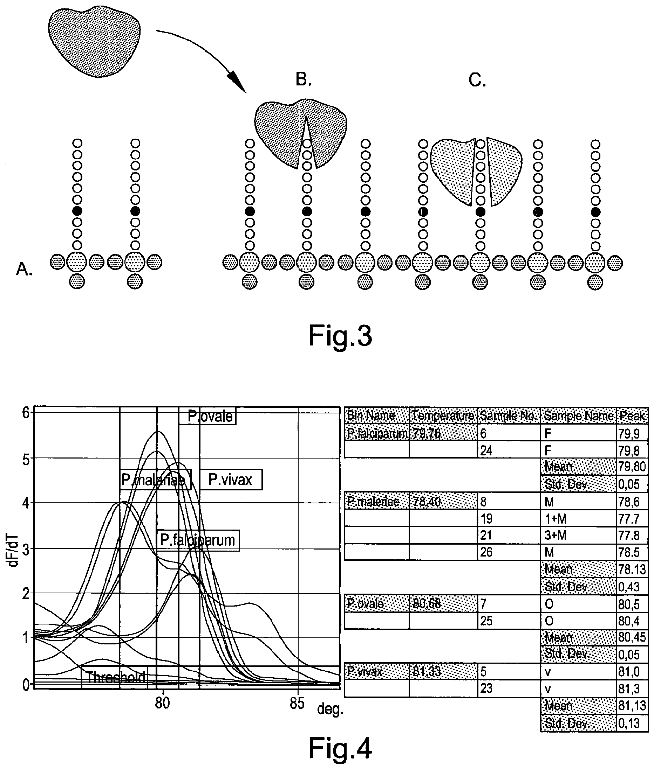

Figure 3 is a schematic diagram showing the method of action of the SiQAC functionalised cellulose. SiQAC chains bind to the cellulose fibres and become oriented and cross-linked (labelled A) to form an active layer. The hydrophobic alkyl chain (labelled B) penetrates the similarly hydrophobic cell wall of the micro-organism. As the alkyl chain penetrates the delicate cell wall, the wall is weakened and punctured. Then the cationic quaternary ammonium group (C) comes in contact with the cell wall, and disrupts the ion flow and causes leakage into or out of the cell wall, usually resulting in the cell losing its contents or, depending on the ionic environment, bursting the cell completely. In its weakened state the rapid drying process of the cells further weakens the cell wall such that during the early cycling of PCR, nucleic acid material is released into the solution and is amplified during the PCR.

Thus, the mode of action of SiQAC molecules involves a number of distinct elements. There is perturbation of cytoplasmic and outer membrane lipid bilayers and cell walls via the alkyl chain, resulting in generalised and progressive leakage of cytoplasmic material resulting in cell lysis, partial or complete depending on ionic strength of the surrounding solution. This is increased by the positively charged ammonium group which associates with negatively charged membrane phospholipid. Even at low concentration of the active components, leakage of low molar mass cytoplasmic components occurs (e.g. K+, nucleic acids and amino acids). SiQAC compounds are considerably more potent than a non-silylated quaternary ammonium compound because the silyl group bonds to surfaces causing the antimicrobial portion to become locally concentrated and orientated.

The process of cell lysis by SiQAC compounds works at room temperature, unlike other processes in the current state of the art. SiQAC functionalised cellulose is suitable to weaken and/or destroy cell walls of bacteria, fungi and protein coat of virus particles. In preferred embodiments, it provides lysed cellular material that is PCR-ready within 5 minutes. The functionalised substrates and methods of the present disclosure enable nucleic acid extraction of DNA and RNA combined with decontamination of other potentially harmful agents within the sample in a single step. It is also possible to extract of RNA from viral particles, providing a single step process negating the need for complex sample processing to access RNA ahead of RT-PCR.

The methods could be used as a means of selecting cell types for lysis e.g. data here shows after 24 hours incubation in blood that whole blood cells remain intact, whilst white erythrocytes become lysed.

We believe that the SiQAC process is compatible with at least the following microorganisms:
Bacteria: MRSA, CA-MRSA; Micrococcus sp.; Staphylococcus epidermis; Enterobacter agglomerans; Acinetobacter calcoaceticus; Staphylococcus aureus (pigmented); Staphylococcus aureus (non-pigmented); Klibsiella pneumonial moniae; Pseudomonas aeruginosa ; Streptococcus faecalis; Escherichia coli; Proteus mirabilis; Citrobacter diversus; Salmonella typhosa; Salmonella choleraesuis; Cornyebacterium bovis; Mycobacterium smegmatis; Mycobacterium tuberculosis; Brucella canis; Brucella abortus; Brucella suis; Streptococcus mutans; Bacillus subtilis; Clostridium perfringens; Haemophilus influenzae; Haemophilus suis; Lactobacillus casel; Leuconostoc lactis; Listeria monocytogenes; Propionibacterium acnes; Proteus vulgaris.
Fungi: Alternaria; Aspergillus flavus; Aspergillus fumigatus; Aspergillus niger; Aspergillus terreus; Aspergillus versicolor; Aureobadisium pullulans; Cephaldascus fragrans; Chaetomium globosum; Cladosporium herbarum; Epidermophyton; Fusarium nigrum; Fusarium solani; Glicocladium roseum; Mucor; Oospora lactis; Pencillium albicans; Tricophyton mentagraphophytes; Pencillium elegans; Pencillium funiculosum; Pencillium humicola; Pencillium notatum; Pullularia pullulans; Penicillium variabile; Rhizopus nigricans; Ricoderm; Stachybotrys atra; Trichophyton interdigitalie; Trichderma flavus; Penicillium citrinum.
Yeast and Algae: Saccharomyces cerevisiae; Candida albicans; Oscillatoria borneti LB143; Anabaena cylindrica; Selenastrum gracile B-325; Pleurococcus LB11; Schenedesmus quadricuada; Gonium LB 9c; Volvox LB 9; Chlorella vulgaris; Cyanophyta (blue-green); Chrysophyta (brown); Chlorophyta (green) Seienastum; Chlorophyta (green) Protococcus.
Viruses; HIV; Dengue; Influenza A/B; SARS; H1N1 (swine flu); H3N2; Herpes Simplex Type 1
Others: Plasmodium malariae.

Details of antimicrobial action of SiQACs can be found in Isquith et al (1972).

### EXAMPLES

The paper used in all examples was the cotton-derived cellulose paper FP 2992 from Hahnemühle FineArt GmbH (Germany), unless otherwise stated.

### Example 1: Paper based purification of bacterial cultures with SiQAC solution and PCR product characterisation

### Protocol:

1. Prepare a O/N culture of a given bacteria (*K. pneumoniae, S. aureus* and *M*. *Tuberculosis**) and take 50µl. [*MTB culture had been incubated for two weeks (O.D. 1.1)]
2. Prepare three sets of cards to be dried O/N
   i. Untreated paper
   ii. Functionalised with 5µl of SiQAC (3-TPAC) solution diluted in 15µl of water
   iii. Functionalised with 5µl of SiQAC (3-TPAC) solution diluted in 15µl of TRIS-CI [pH 7.5]
3. Add 20µl of the cultures to each set of treated and untreated paper and dry at room temperature for 10 minutes [x4 repeats on different paper for each culture]
4. Remove a paper-punched disk using a sterile biopsy punch (1.5mm).
5. Add the punched disk to the PCR mix (20µl final volume)
6. Carry out 35 cycles of 16S universal for each of the tests; *K. pneumoniae* and *S. aureus,* GD's MTB/RIF
7. Use standard pyrosequencing of PCR products for *K. pneumoniae* and *S. aureus*

### Results:

1. PCR from std pads (set 1) for *K. pneumoniae* yielded an average 18 high quality bp readouts in pyro
2. PCR from pads enriched with 5µl of SiQAC (3-TPAC) solution added to 15µl of water (set 2) for *K. pneumoniae* yielded an average 24 medium quality bp readouts in pyro
3. PCR from pads enriched with 5µl of SiQAC (3-TPAC) solution added to 15µl of TRIS-CI [pH 7.5] (set 3) for *K. pneumoniae* yielded an average 28 high quality bp readouts in pyro
4. PCR from untreated pads (set 1) for *S. aureus* yielded an average 16 high quality bp readouts in pyro
5. PCR from pads enriched with 5µl of SiQAC (3-TPAC) solution added to 15µl of water (set 2) for *S.aureus* yielded an average 20 medium quality bp readouts in pyro
6. PCR from pads enriched with 5µl of SiQAC (3-TPAC) solution added to 15µl of TRIS-CI [pH 7.5] (set 3) for *S. aureus* yielded an average 25 high quality bp readouts in pyro
7. GD's MTB/RIF: MTB identification was correct for the three sets of samples, including RIF identification in samples processed through pads enriched with 5µl of SiQAC (3-TPAC) solution diluted with 15µl of TRIS-CI [pH 7.5]

### Conclusions:

Paper enriched with SiQAC (3-TPAC) solution successfully lysed the bacterial cells allowing PCR from the extracted nucleic acid. This was confirmed by the raw length of sequence obtained through pyrosequencing, and the end-point detection of MTB-RIF from Mycobacterium tuberculosis which is a difficult cell to break open, conventionally requiring harsh treatments with NALC-NaOH to achieve the same level of cell disruption. Untreated paper did not yield the same results. TRIS-CI [pH 7.5] is needed to buffer the SiQAC.

### Example 2: Detection of Plasmodium species from blood treated with SiQAC solution.

Figure 4 shows the results of detection of nucleic acids from different Plasmodium species in blood samples obtained from infected patients.

### Example 3: Paper functionalisation and use methods

Three alternative paper functionalisation methods are given:

### Process A

Paper functionalised with 20µl SiQAC (3-TPAC) solution, dried for 24 hours and then washed in 20µl 5mM TRIS-Cl [pH 9.0], 0.2mM MgCl2 then dried and either;
i. Apply the sample, dry for 20', remove disc and add 20µl of miliQ water for PCR, or
ii. Apply the sample, dry for 20', remove disc and rinse in 20µl of miliQ water using gentle pipetting, use 20µl in PCR

### Process B

Paper functionalised with 20µl SiQAC (3-TPAC) solution, dried for 30' and washed in 5mM TRIS-CI [pH 9.0], 0.2mM MgCl2 then dried and either;
i. Apply the sample, dry for 20', remove disc and add 20µl of miliQ water for PCR, or
ii. Apply the sample, dry for 20', remove disc and rinse in 20µl of miliQ water using gentle pipetting, use 20µl in PCR

### Process C

Paper functionalised with 20µl SiQAC (3-TPAC) and dried for 30' and either;
i. Apply the sample, dry for 20', remove disc and rinse in 20µl of miliQ water using gentle pipetting, use 20µl in PCR, or
ii. Apply the sample, dry for 20', remove disc and add 20µl of 5mM TRIS-CI [pH 9.0], 0.2mM MgCl2 for PCR; or
iii. Apply the sample, dry for 20', remove disc and rinse in 20µl of 5mM TRIS-CI [pH 9.0], 0.2mM MgCl2 using gentle pipetting, use 20µl in PCR

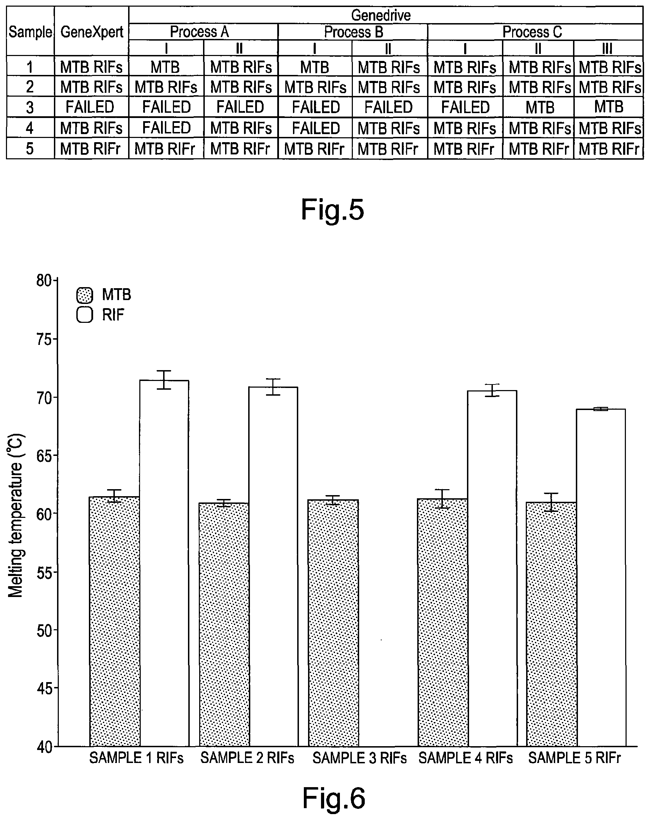

The various different paper processing methods were compared for extraction of Mycobacterium tuberculosis DNA from clinical samples of raw sputum and results obtained using the standard Cepheid GeneXpert® MTB processing using NALC-NaOH. The results are shown in Figure 5. (MTB = Mycobacterium rpoB gene detected; RIFs or RIFr = rifampicin mutations detected; FAILED = no detection). The three processing methods released nucleic acids to varying degree into solution for PCR allowing detection of the multi-copy and single copy rpoB gene and to detect the rifampicin mutation. All processes gave comparative data to the GeneXpert® gold standard system. Process C gave 'better than' results in comparison to the gold standard, giving a high degree of cellular disruption and stability of high quality DNA for the PCR analysis, further confirmed by pyrosequencing. Pyrosequencing showed sequence variations observed in 30bp of sequence from amplicons from the V2 ribosomal DNA, long yellow & blue reads illustrate the absence of mutations in the sequence confirming the presence of good, amplification products.

Figure 6 shows the cumulative accuracy data from 5 clinical sputum samples (4x RIFs and 1x RIFr), processed using the 7 different modifications described (i.e. 35 tests). Measurement of melt temperatures for MTB (light blue) and rpoB mutations indicating RIF status (RIFs, RIFr) and associated error bars representing standard deviation across the data points measured show that the process does not influence the accuracy of the melt peak determination which is within ± 0.5°C. Standard methodologies can influence accuracy of measurement.

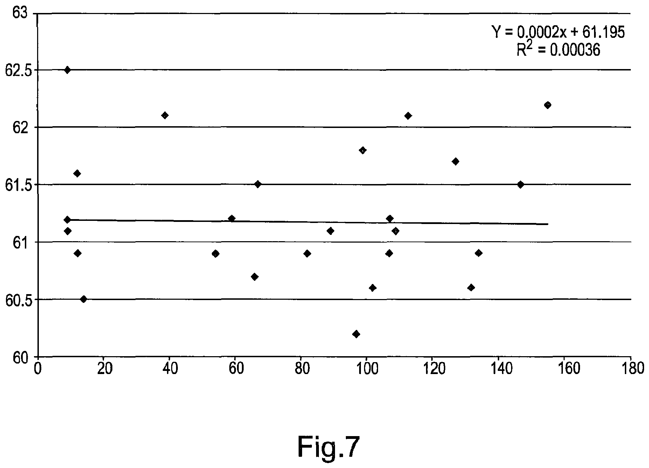

Figure 7 shows multiple repeat tests of MTB at different concentrations extracted from sputa using the process described, the y axis showing the peak melt temperature and the x axis showing the peak height.

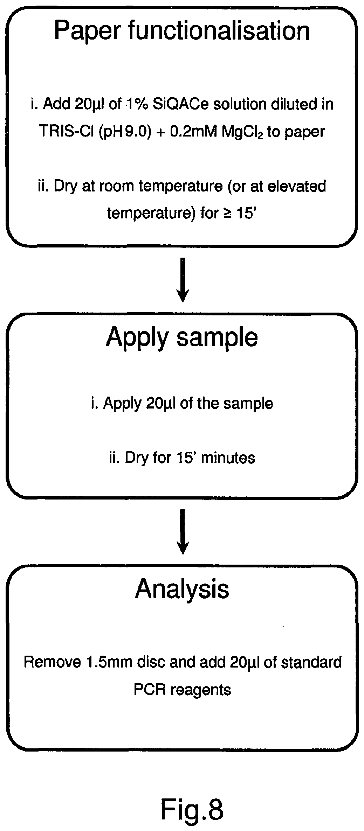

In light of these examples, Figure 8 shows the optimised process for paper functionalisation with SiQAC compounds and sample analysis.

### Example 4. Pyrosequencing of Mycobacterium tuberculosis extracted using SiQAC from sputum samples

Three sputum samples were processed using SiQAC (3-TPAC) functionalised composite cards. 1.5mm discs were added to a standard 16S ribosomal DNA amplification with no hot start (i.e. cycling only) and the resulting amplicons were analysed using pyrosequencing. The results showed good amplification of core sequence regions providing >25 nucleotide read lengths indicative of good extraction and amplification:
Search mode: Full search
Mean identity score: 100%
Search engine: PyroMark Q96 ID
Reference database: HULPII
Reference sequence: M. microti ATCC19422.
Sample 1 > CGGCTGCTGGCACGTAGTTGGCCGGTCCTTCTT
Sample 2 > CGGCTGCTGGCACGTAGTTGGCCGGTCCTTCTT
Sample 3 > CGGCTGCTGGCACGTAGTTGGCCGGTCC

### Example 5: Isolation of microbial DNA from whole milk

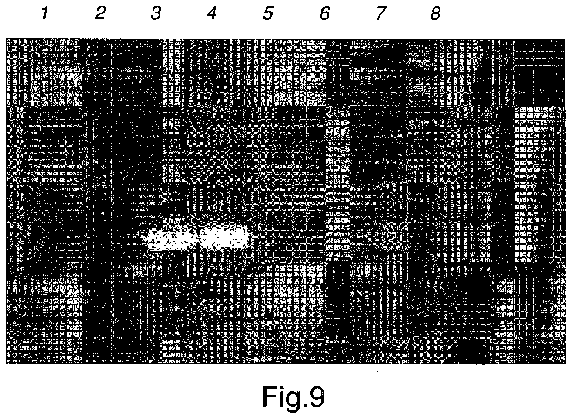

Whole milk is a difficult sample type for PCR with many inhibitors. Composite paper was functionalised using the preferred method (illustrated in Figure 8). A single 1.5mm disc of functionalised paper plus dried milk sample was added to a standard PCR mix with primers for universal 16S ribosomal DNA amplification. Amplified DNA was run on a standard gel. Figure 9 shows the results. (Lane 1: Molecular weight marker; Lane 2: 5µl E. coli reference gDNA + 1µl 100% Si-QAC (3-TPAC) solution; Lane 3: 5µl E. coli reference gDNA + 1µl 10% Si-QAC (3-TPAC) solution; Lane 4: 5µl E. coli reference gDNA + 1µl 1% Si-QAC (3-TPAC) solution; Lane 5: 1.5mm disc functionalised with 10% Si-QAC (3-TPAC) solution + 20µl of whole dairy milk; Lane 6: 1.5mm disc functionalised with 1% Si-QAC (3-TPAC) solution + 20µl of whole dairy milk; Lane 7: 1.5mm disc functionalised + 20µl of whole dairy milk; Lane 8: 1µl whole dairy milk).

The data shown demonstrates that functionalisation with 1% Si-QAC (3-TPAC) enhances amplification compared to untreated paper (lanes 6 & 7).

Discs were checked for residual bacteria by inoculating enrichment media for lactic bacteria (tryptone 5g/l, dextrose 1g/l, yeast extract 2.5g/l and skimmed milk powder 1g/l) plus 4% agar - R.C. MARSHALL (1993) Standard Methods for the Microbiological examination of dairy products, 16th Ed. (American Public Health Association). Overnight culture showed no bacterial growth on discs treated with Si-QAC (3-TPAC) but confluent growth on culture plates of untreated discs confirming that the functionalised paper decontaminated the liquid within 15 minutes.

### Example 6: rt-pcr from RNA extracted from HIV virus from serum using functionalised cards

### SUMMARY

Functionalised cards worked for RT-nested PCR of HIV K103 amplicon with a plasma sample from a positively diagnosed anonymous patient. Parallel assay with a full blood sample from a different patient (also positively diagnosed) gave no results either in PCR (no fragments were detected in 1% agarose gels) or pyro.

### ASSAY DESCRIPTION

Two cards were functionalized using purification solution (PF solution, containing 3-TPAC) following the standardized protocol:
1. Open the card and apply 20 microl. of 1/100 PF solution diluted in buffer (5 mM Tris HCl (pH 9.0), 0'2 mM MgCl2)
2. Dry for 15 minutes
3. Apply 20 microl. of sample*
4. Dry for 15 minutes
5. Punch a single disc and add to RT

*Samples were plasma from peripheral HIV positive blood (1) and peripheral HIV positive blood (2)

RT mix used was Applied Biosystems Superscript VILO cDNA synthesis kit as described in the user's manual adjusting to a final volume of 30microl.
PCR was later prepared in the following way:
H20 - 5.8 µl
10x PCR buffer - 5 µl
MgCl2 [50mM] - 2 µl
dNTPs •10mM• - 1.2 µl
1849+ •10µM• - 1 µl
3500- •10µM• - 1 µl
Ultratools Taq polymerase [1u/µl] - 1 µl
cDNA from Superscript VILO cDNA synthesis kit - 30 µl

**Primers used were:**

| | |
|---|---|
| 1849+ | 5'-GATGACAGCATGTCAGGGAG |
| 3500- | 5'-CTATTAAGTATTTTGATGGGTCATAA |

**PCR program:**

| | |
|---|---|
| 94°C | 2 min, followed by 45 cycles of |
| 94°C | 30 sec |
| 50°C | 30 sec |
| 72°C | 1 min 30 sec ; followed by |
| 72°C | 5min |
| 10°C | ∞ |

The product of this PCR is 1702 bp long and contains the gag-pro-pol region of HIV1. This amplicon was used as a template for the second PCR.

Second PCR was prepared in the following way:
H2O - 20 µl
10x PCR buffer - 4 µl
MgCl2 [50mM] - 1.6 µl
dNTPs •10mM• - 0.8 µl
K103F •10µM• - 3.2 µl
BioK103F •10µM• - 3.2 µl
Biotools Taq polymerase [1 u/µl] - 0.5 µl
PCR product from the nested PCR - 5 µl

**Primers used were:**

| | |
|---|---|
| K193F | 5'-GGAATACCACATCCYGCAGG |
| BioK103R | 5'-[Biotin]AATATTGCTGGTGATCCTTTCC |

**PCR program:**

| | |
|---|---|
| 95°C | 5 min; followed by 30 cycles of |
| 95°C | 30 sec |
| 55°C | 30 sec |
| 72°C | 30 sec ; followed by |
| 72°C | 5min |
| 10°C | ∞ |

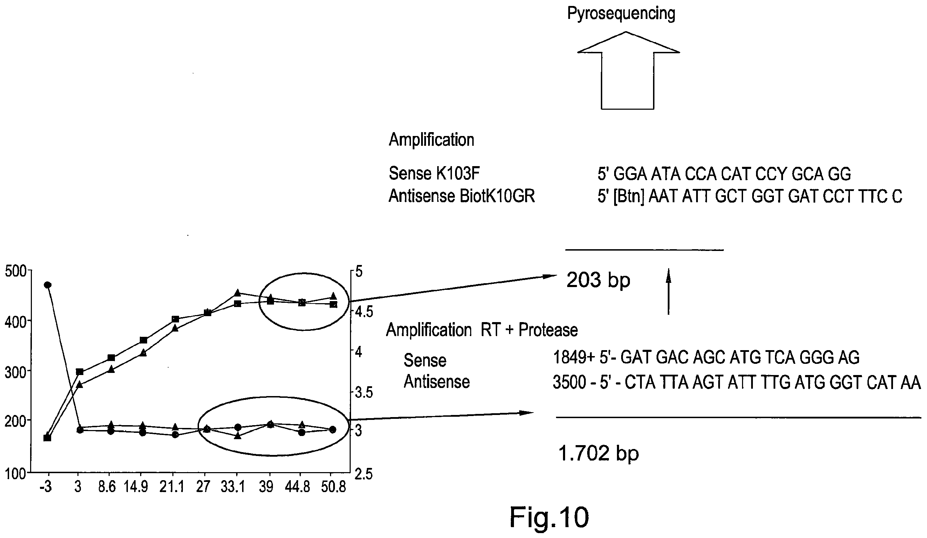

The PCR product from the second PCR is 203bp and was used in the pyrosequencing reaction following the enrichment approach shown in Figure 10.

### RESULTS

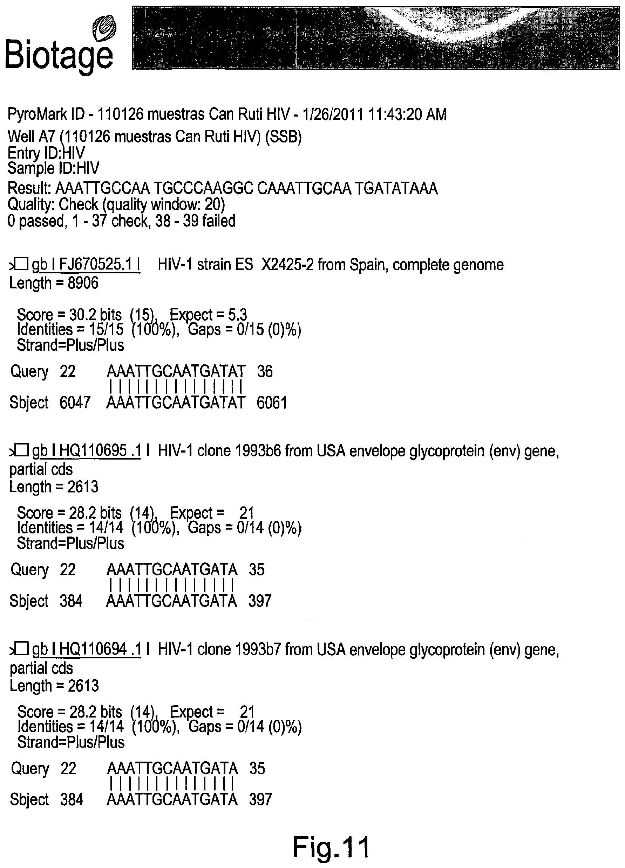

Only the plasma sample gave good signals in the pyrosequencer, the blood sample gave no result at all following the protocol described. The sequencing primer used was K103F (5'-GGAATACCACATCCYGCAGG) and the sequence obtained was matched against the complete HIV genome using the BLAST tool of the NCBI. The sequence successfully matched to the HIV - see Figure 11.

## Claims

1. A method of preparing nucleic acids from a biological sample comprising a) cellular material having a cell membrane, or b) cellular material having a cell wall, or c) viral material having a viral envelope or d) viral material having a viral capsid; the method comprising contacting the sample with a substrate, the substrate being functionalised with a biocidal agent which is capable of i) weakening the cell membrane, cell wall, viral envelope, or viral capsid; or ii) lysing cellular or viral material; wherein the biocidal agent comprises multiple functional groups, the multiple functional groups comprising a silyl group, which is involved in binding the agent to the substrate; an alkyl chain; and an ammonium chloride group.

2. The method of claim 1, further comprising the step of subjecting the sample to heat after the contacting step.

3. The method of claim 1 or 2, wherein a) the substrate is a cellulose material; preferably a cellulose filter paper or a cellulose matrix; more preferably wherein the cellulose material is a composite paper; most preferably wherein the composite paper comprises a lateral flow layer, to remove liquid and low molecular weight contaminants and inhibitors from the sample which is deposited on a surface of the paper; or b) wherein the substrate is glass or plastic.

4. The method of any preceding claim wherein the substrate is in the form of a microfluidic channel or a reaction vessel or other container.

5. The method of any preceding claim wherein the alkyl chain is C5-C30 alkyl, preferably C10-C20 alkyl.

6. The method of any preceding claim wherein the biocidal agent is a silylated quaternary ammonium compound (SiQAC); more preferably wherein the biocidal agent is 3-(trimethoxysilyl) propyldimethyloctadecyl ammonium chloride

7. The method of any preceding claim
i. wherein a) the biological sample comprises cellular material having a cell wall; b) the biological sample comprises eukaryotic animal cells; or c) the biological sample comprises viral material; or
ii. wherein the cellular material comprises a prokaryotic cell, or wherein the cellular material comprises plant or fungal cells.

8. A method of amplifying nucleic acids in a biological sample, the method comprising: preparing nucleic acids according to the method of any of claims 1 to 7; and subjecting the prepared acids to a nucleic acid amplification step, preferably a polymerase chain reaction (PCR) amplification.

9. A device for preparing nucleic acids from a biological sample comprising a) cellular material having a cell membrane, or b) cellular material having a cell wall, or c) viral material having a viral envelope or d) viral material having a viral capsid; the device comprising a substrate functionalised with a biocidal agent which is capable of i) weakening the cell membrane, cell wall, viral envelope, or viral capsid; or ii) lysing cellular or viral material, wherein the biocidal agent comprises multiple functional groups, the multiple functional groups comprising a silyl group, which is involved in binding the agent to the substrate; an alkyl chain; and an ammonium chloride group.

10. The device of claim 9, wherein the substrate is a) a cellulose material; preferably a cellulose filter paper or a cellulose matrix; more preferably wherein the cellulose material is a composite paper; or b) is glass or plastic.

11. The device of any of claims 9 or 10 wherein the substrate is in the form of a microfluidic channel or a reaction vessel or other container.

12. The device of any of claims 9-11 wherein the alkyl chain is C5-C30 alkyl, preferably C10-C20 alkyl.

13. The device of any of claims 9-12 wherein the biocidal agent is a silylated quaternary ammonium compound (SiQAC); more preferably wherein the biocidal agent is 3-(trimethoxysilyl) propyldimethyloctadecyl ammonium chloride.

14. The device of any of claims 9 to 13 wherein the substrate is integrated into a sample preparation cartridge or the like.

## Patentansprüche

1. Verfahren zum Herstellen von Nukleinsäuren aus einer biologischen Probe, umfassend a) zelluläres Material mit einer Zellmembran oder b) zelluläres Material mit einer Zellwand oder c) virales Material mit einer viralen Hülle oder d) virales Material mit einem viralen Kapsid; wobei das Verfahren Inkontaktbringen der Probe mit einem Substrat umfasst, wobei das Substrat mit einem bioziden Mittel funktionalisiert ist, welches zum i) Schwächen der Zellmembran, Zellwand, viralen Hülle oder des viralen Kapsids; oder ii) Lysieren von zellulärem oder viralem Material fähig ist; wobei das biozide Mittel mehrfache funktionelle Gruppen umfasst, wobei die mehrfachen funktionellen Gruppen eine Silylgruppe, welche an der Bindung des Mittels an das Substrat beteiligt ist; eine Alkylkette; und eine Ammoniumchloridgruppe umfassen.

2. Verfahren nach Anspruch 1, weiterhin umfassend den Schritt, die Probe nach dem Schritt des Inkontaktbringens Wärme auszusetzen.

3. Verfahren nach Anspruch 1 oder 2, wobei a) das Substrat ein Cellulosematerial; vorzugsweise ein Cellulosefilterpapier oder eine Cellulosematrix, ist; stärker bevorzugt, wobei das Cellulosematerial ein Verbundpapier ist; am meisten bevorzugt, wobei das Verbundpapier eine Schicht mit lateralem Fluss umfasst, um Flüssigkeit und Kontaminanten und Inhibitoren mit niedrigem Molekulargewicht aus der Probe zu entfernen, welche auf einer Oberfläche des Papiers abgeschieden wird; oder b), wobei das Substrat Glas oder Kunststoff ist.

4. Verfahren nach einem vorhergehenden Anspruch, wobei das Substrat in der Form von einem mikrofluidischen Kanal oder einem Reaktionsgefäß oder anderen Behälter ist.

5. Verfahren nach einem vorhergehenden Anspruch, wobei die Alkylkette C5-C30-Alkyl, vorzugsweise C10-C20-Alkyl, ist.

6. Verfahren nach einem vorhergehenden Anspruch, wobei das biozide Mittel eine silylierte quaternäre Ammoniumverbindung (SiQAC) ist; stärker bevorzugt, wobei das biozide Mittel 3-(Trimethoxysilyl)propyldimethyloctadecylammoniumchlorid ist.

7. Verfahren nach einem vorhergehenden Anspruch
i. wobei a) die biologische Probe zelluläres Material mit einer Zellwand umfasst; b) die biologische Probe eukaryotische tierische Zellen umfasst; oder c) die biologische Probe virales Material umfasst; oder
ii. wobei das zelluläre Material ein prokaryotische Zelle umfasst oder wobei das zelluläre Material Pflanzen- oder Pilzzellen umfasst.

8. Verfahren zum Amplifizieren von Nukleinsäuren in einer biologischen Probe, wobei das Verfahren umfasst: Herstellen von Nukleinsäuren gemäß dem Verfahren nach einem der Ansprüche 1 bis 7; und Unterwerfen der hergestellten Säuren einem Nukleinsäureamplifikationsschritt, vorzugsweise einer Polymerasekettenreaktions-(PCR)-Amplifikation.

9. Vorrichtung zum Herstellen von Nukleinsäuren aus einer biologischen Probe, umfassend a) zelluläres Material mit einer Zellmembran oder b) zelluläres Material mit einer Zellwand oder c) virales Material mit einer viralen Hülle oder d) virales Material mit einem viralen Kapsid; wobei die Vorrichtung ein Substrat, funktionalisiert mit einem bioziden Mittel, umfasst, welches zum i) Schwächen der Zellmembran, Zellwand, viralen Hülle oder des viralen Kapsids; oder ii) Lysieren von zellulärem oder viralem Material fähig ist, wobei das biozide Mittel mehrfache funktionelle Gruppen umfasst, wobei die mehrfachen funktionellen Gruppen eine Silylgruppe, welche an der Bindung des Mittels an das Substrat beteiligt ist; eine Alkylkette; und eine Ammoniumchloridgruppe umfassen.

10. Vorrichtung nach Anspruch 9, wobei das Substrat a) ein Cellulosematerial; vorzugsweise ein Cellulosefilterpapier oder eine Cellulosematrix, ist; stärker bevorzugt, wobei das Cellulosematerial ein Verbundpapier ist; oder b) Glas oder Kunststoff ist.

11. Vorrichtung nach einem der Ansprüche 9 oder 10, wobei das Substrat in der Form von einem mikrofluidischen Kanal oder einem Reaktionsgefäß oder anderen Behälter ist.

12. Vorrichtung nach einem der Ansprüche 9-11, wobei die Alkylkette C5-C30-Alkyl, vorzugsweise C10-C20-Alkyl, ist.

13. Vorrichtung nach einem der Ansprüche 9-12, wobei das biozide Mittel eine silylierte quaternäre Ammoniumverbindung (SiQAC) ist; stärker bevorzugt, wobei das biozide Mittel 3-(Trimethoxysilyl)propyldimethyloctadecylammoniumchlorid ist.

14. Vorrichtung nach einem der Ansprüche 9 bis 13, wobei das Substrat in eine Probenaufbereitungskartusche oder dergleichen integriert ist.

## Revendications

1. Méthode pour la préparation d'acides nucléiques à partir d'un échantillon biologique comprenant a) un matériau cellulaire possédant une membrane cellulaire, ou b) un matériau cellulaire possédant une paroi cellulaire, ou c) un matériau viral possédant une enveloppe virale, ou d) un matériau viral possédant une capside virale; la méthode comprenant la mise en contact de l'échantillon avec un substrat, le substrat étant fonctionnalisé avec un agent biocide qui est capable i) d'affaiblir la membrane cellulaire, la paroi cellulaire, l'enveloppe virale ou la capside virale; ou ii) de lyser le matériau cellulaire ou viral; dans laquelle l'agent biocide comprend des groupes fonctionnels multiples, les groupes fonctionnels multiples comprenant un groupe silyle, qui est impliqué dans la liaison de l'agent au substrat; une chaîne alkyle; et un groupe de chlorure d'ammonium.

2. Méthode selon la revendication 1, comprenant en outre l'étape d'exposition de l'échantillon à de la chaleur après l'étape de mise en contact.

3. Méthode selon la revendication 1 ou 2, dans laquelle a) le substrat est un matériau de cellulose; de préférence un papier filtre de cellulose ou une matrice de cellulose; plus préférablement dans laquelle le matériau de cellulose est un papier composite; le plus préférablement dans laquelle le papier composite comprend une couche de circulation latérale, pour retirer le liquide et les contaminants et inhibiteurs à bas poids moléculaire à partir de l'échantillon qui est déposé sur une surface du papier; ou dans laquelle b) le substrat est du verre ou du plastique.

4. Méthode selon l'une quelconque des revendications précédentes, dans laquelle le substrat est sous la forme d'un canal microfluidique ou d'une cuve de réaction ou d'un autre récipient.

5. Méthode selon l'une quelconque des revendications précédentes, dans laquelle la chaîne alkyle est un C5-C30 alkyle, de préférence un C10-C20 alkyle.

6. Méthode selon l'une quelconque des revendications précédentes, dans laquelle l'agent biocide est un composé d'ammonium quaternaire silylé (SiQAC); plus préférablement dans laquelle l'agent biocide est le chlorure de 3-(triméthoxysilyl)propyldiméthyloctadécylammonium.

7. Méthode selon l'une quelconque des revendications précédentes:
i. dans laquelle a) l'échantillon biologique comprend un matériau cellulaire possédant une paroi cellulaire; b) l'échantillon biologique comprend des cellules eucaryotes animales; ou c) l'échantillon biologique comprend un matériau viral; ou
ii. dans laquelle le matériau cellulaire comprend une cellule procaryote ou dans laquelle le matériau cellulaire comprend des cellules végétales ou fongiques.

8. Méthode pour l'amplification d'acides nucléiques dans un échantillon biologique, la méthode comprenant: la préparation d'acides nucléiques selon la méthode de l'une quelconque des revendications 1 à 7; et l'exposition des acides préparés à une étape d'amplification d'acides nucléiques, de préférence une amplification par réaction en chaîne de la polymérase (PCR).

9. Dispositif pour la préparation d'acides nucléiques à partir d'un échantillon biologique comprenant a) un matériau cellulaire possédant une membrane cellulaire, ou b) un matériau cellulaire possédant une paroi cellulaire, ou c) un matériau viral possédant une enveloppe virale, ou d) un matériau viral possédant une capside virale; le dispositif comprenant un substrat fonctionnalisé avec un agent biocide qui est capable i) d'affaiblir la membrane cellulaire, la paroi cellulaire, l'enveloppe virale ou la capside virale; ou ii) de lyser le matériau cellulaire ou viral; dans lequel l'agent biocide comprend des groupes fonctionnels multiples, les groupes fonctionnels multiples comprenant un groupe silyle, qui est impliqué dans la liaison de l'agent au substrat; une chaîne alkyle; et un groupe de chlorure d'ammonium.

10. Dispositif selon la revendication 9, dans lequel le substrat est a) un matériau de cellulose; de préférence un papier filtre de cellulose ou une matrice de cellulose; plus préférablement dans lequel le matériau de cellulose est un papier composite; ou b) du verre ou du plastique.

11. Dispositif selon l'une quelconque des revendications 9 et 10, dans lequel le substrat est sous la forme d'un canal microfluidique ou d'une cuve de réaction ou d'un autre récipient.

12. Dispositif selon l'une quelconque des revendications 9-11, dans lequel la chaîne alkyle est un C5-C30 alkyle, de préférence un C10-C20 alkyle.

13. Dispositif selon l'une quelconque des revendications 9-12, dans lequel l'agent biocide est un composé d'ammonium quaternaire silylé (SiQAC); plus préférablement dans laquelle l'agent biocide est le chlorure de 3-(triméthoxysilyl)propyldiméthyloctadécylammonium.

14. Dispositif selon l'une quelconque des revendications 9 à 13, dans lequel le substrat est intégré dans une cartouche de préparation d'échantillon ou équivalent.
